(19) Europäisches Patentamt
European Patent Office
Office européen des brevets

(11) EP 1 375 483 A1

(12) **EUROPEAN PATENT APPLICATION**
published in accordance with Art. 158(3) EPC

(43) Date of publication:
**02.01.2004 Bulletin 2004/01**

(21) Application number: **01912233.2**

(22) Date of filing: **09.03.2001**

(51) Int Cl.7: **C07D 213/61**, C07D 405/12, A01N 43/40, A01N 47/00

(86) International application number:
**PCT/JP2001/001866**

(87) International publication number:
**WO 2001/066523 (13.09.2001 Gazette 2001/37)**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **KUREHA KAGAKU KOGYO KABUSHIKI KAISHA**
**Chuo-ku, Tokyo 103-8552 (JP)**

(72) Inventors:
- **ITO, Atsushi**
  **Iwaki-shi, Fukushima 974-8232 (JP)**
- **SUDO, Keiichi**
  **Iwaki-shi, Fukushima 974-8232 (JP)**
- **WATANABE, Tsumoru**
  **Kitaibaraki-shi, Ibaraki 319-1704 (JP)**
- **EIZUKA, Takayoshi**
  **Iwaki-shi, Fukushima 974-8232 (JP)**
- **NIIZEKI, Yoshitaka**
  **Iwaki-shi, Fukushima 974-8232 (JP)**

(74) Representative: **Hayes, Adrian Chetwynd et al**
**Boult Wade Tennant,**
**Verulam Gardens**
**70 Gray's Inn Road**
**London WC1X 8BT (GB)**

(54) **6-CHLORO-3-PYRIDYLMETHYLPROPYLAMINE DERIVATIVES, PREPARATION PROCESS THEREOF AND BACTERICIDES**

(57) A 6-chloro-3-pyridylmethylpropylamine derivative according to the present invention or an acid addition salt thereof is represented by the following formula (I):

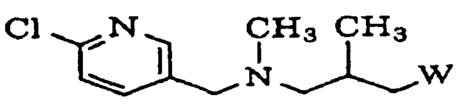

(I)

wherein W represents 3,5-bistrifluoromethylphenyl group, 4-trifluoromethoxyphenyl group, 3-chloro-5-fluorophenyl group, 2,2-difluoro-1,3-benzodioxol-5-yl group and the like.

EP 1 375 483 A1

## Description

### Technical Field

The present invention relates to novel propylamine derivatives that have physiological activities, the preparation method and fungicides, and more particularly, novel propylamine derivatives that are fit to use as agricultural and horticultural fungicides, the preparation method and fungicides that contain the derivatives as an effective ingredient.

### Background Art

3-phenylpropylamines such as N-[3-p-t-butylphenyl-2-methyl-1-propyl]-cis-2,6-dimethy lmorpholine (fenpropimorph) described in Japanese Unexamined Patent Publication No. SHO 53-77070 and N-[3-p-t-butylphenyl-2-methyl-1-propyl] piperidine (fenpropidine) described in Japanese Unexamined Patent Publication No. SHO 53-68785 and Japanese Unexamined Patent Publication No. SHO 53-68786 are commercially available as fungicides.

Although nitrogen atoms of the amino groups in the foregoing compounds form a part of the ring structures, certain compounds are known that nitrogen atoms of the amino groups do not form the part of the ring structures and a heterocyclicmethyl group is bonded to the nitrogen atom, whose examples are the compounds described in Japanese Unexamined Patent Publication No. SHO 63-258867, wherein the compounds have a heterocyclicmethyl group such as a tetrahydrofurfuryl group and a thenyl group containing oxygen and sulfur, respectively, and the following N-heterocyclicmethylpropylamine derivatives that are described in the literature Pestic. Sci., 35, 339 (1992).,
N-[3-(4-t-butylphenyl)-2-methylpropyl]-N-(t-butyl)-3-py ridylmethylamine,
N-[3-(4-t-butylphenyl)-2-methylpropyl]-N-butyl-3-pyridy lmethylamine and
N-[3-(4-t-butylphenyl)-2-methylpropyl]-N-methyl-3-pyrid ylmethylamine.

Additionally, Publication No. WO99/12902 discloses the N-heterocyclicmethylpropylamine derivatives, and it is described that the fungicide having the above derivatives as an effective ingredient has a control effect on plant diseases.

However, even the conventional N-heterocyclicmethylpropylamine derivatives described in the aforementioned Publication No. WO99/12902 are yet insufficient to obtain a fungicide that exhibits a highly enough control effect.

### Disclosure of the Invention

It is an object of the present invention to provide novel 6-chloro-3-pyridylmethylpropylamine derivatives that are fit to use as agricultural and horticultural fungicides having a high fungicidal activity on plant diseases and consequently exhibit a remarkably excellent control effect on plant diseases in addition to the low toxicity to men and animals and the high safety for handling, and also to provide the preparation method of 6-chloro-3-pyridylmethylpropylamine derivatives and the fungicides containing them as an effective ingredient.

As a result of assiduous study to accomplish the aforementioned object, thepresent inventors came to achieve the present invention by finding out that the above issue could be overcome because 6-chloro-3-pyridylmethylpropylamine derivatives having a specific structure have a strong fungicidal activity against pathogens of plant diseases and thus exhibit a remarkably excellent control effect on plant diseases utilizing'them as an effective ingredient of the fungicides.

Thus, 6-chloro-3-pyridylmethylpropylamine derivatives of the present invention are those represented by the following formula (I) or the acid addition salts thereof:

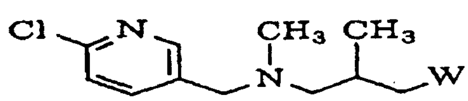

( I )

wherein W is a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group, 3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group, 2,3,5,6-tetrafluoro-4-trifluoromethylphenyl group, 4-trifluoromethoxyphenyl group, 4-difluoromethoxyphenyl group, 4-(1,1,2,3,3,3-hexafluoropropoxy) phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group, 3,5-difluoro-4-trifluoromethoxyphenyl group, 3-chloro-5-fluorophenyl group, 3,5-dichloro-4-fluorophenyl group, pentafluorophenyl group, 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl group and 2,2,4,4-tetrafluoro-4H-1,3- benzodioxin-6-yl group.

The first preparation method of 6-chloro-3-pyridylmethylpropylamine derivatives of the present invention is to employ a reductive amination using 3-phenylpropionaldehyde derivatives represented by the following formula (II):

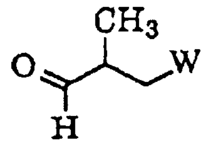

(II)

and 6-chloro-3-pyridylmethylamine derivatives represented by the following formula (III):

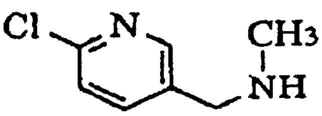

(III)

to obtain the 6-chloro-3-pyridylmethylpropylamine derivatives represented by the following formula (I):

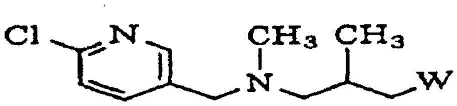

(I)

in the formulae (I) and (II), W represents a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group, 3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group, 2,3,5,6-tetrafluoro-4-trifluoromethylphenyl group, 4-trifluoromethoxyphenyl group, 4-difluoromethoxyphenyl group, 4-(1,1,2,3,3,3-hexafluoropropoxy) phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group, 3,5-difluoro-4-trifluoromethoxyphenyl group, 3-chloro-5-fluorophenyl group, 3,5-dichloro-4-fluorophenyl group, pentafluorophenyl group, 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl group, and 2,2,4,4-tetrafluoro-4H-1,3-benzodioxin-6-yl group.

Furthermore, the second preparation method of 6-chloro-3-pyridylmethylpropylamine derivatives of the present invention is to employ a reductive amination using 3-phenylpropionaldehyde derivatives represented by the following formula (II):

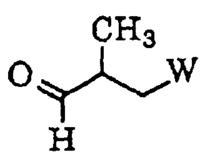

(II)

and 6-chloro-3-pyridylmethylamine derivatives represented by the following formula (IV):

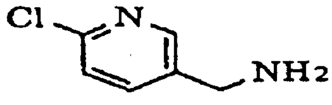

(IV)

to obtain 3-phenylpropylamine derivatives represented by the following formula (V):

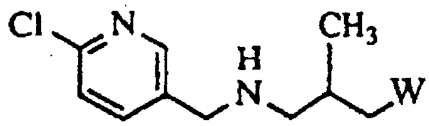

(V)

and then to react the aforementioned 3-phenylpropylamine derivatives with at least one methylating agent selected from the group consisting of formaldehyde and methane derivatives represented by the following formula (VI):

$$CH_3X \qquad (VI)$$

finally resulting in 6-chloro-3-pyridylmethylpropylamine derivatives represented by the following formula (I):

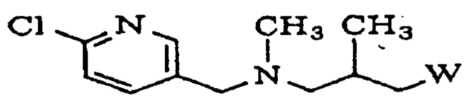

( I )

in the formulae (I), (II) and (V), W represents a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group, 3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group, 2,3,5,6-tetrafluoro-4-trifluoromethylphenyl group, 4-trifluoromethoxyphenyl group, 4-difluoromethoxyphenyl group, 4-(1,1,2,3,3,3-hexafluoropropoxy) phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group, 3,5-difluoro-4-trifluoromethoxyphenyl group, 3-chloro-5-fluorophenyl group, 3,5-dichloro-4-fluorophenyl group, pentafluorophenyl group, 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl. group and 2,2,4,4-tetrafluoro-4H-1,3- benzodioxin-6-yl group, and X in the formula (VI) represents a leaving group.

Additionally, the fungicides in thepresent invention are characterized by containing 6-chloro-3-pyridylmethylpropylamine derivatives represented by the following formula (I) or their acid addition salts as an effective ingredient:

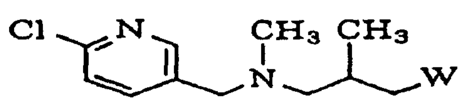

( I )

wherein W represents a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group,

3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group, 2,3,5,6-tetrafluoro-4-trifluoromethylphenyl group, 4-trifluoromethoxyphenyl group, 4-difluoromethoxyphenyl group, 4-(1,1,2,3,3,3-hexafluoropropoxy) phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group, 3,5-difluoro-4-trifluoromethoxyphenyl group, 3-chloro-5-fluorophenyl group, 3,5-dichloro-4-fluorophenyl group, pentafluorophenyl group, 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl group and 2,2,4,4-tetrafluoro-4H-1,3-benzodioxin-6-yl group.

## Brief Description of the Drawings

Each of Figs. 1A to 1D shows a structure of W in the formula (I) which is held by the compounds I 1 to 27 in the present invention.

Fig. 2 shows structures of the comparative compounds (a) to (d) , and (m-1) to (m-3) , which are used in the examples.

## Best Mode for Carrying Out the Invention

The following detailed description relates to a preferred embodiment of the present invention.

(Propylamine derivatives)

The novel 6-chloro-3-pyridylmethylpropylamine derivatives of the present invention are 6-chloro-3-pyridylmethylpropylamine derivatives represented by the following formula (I) or their acid addition salts:

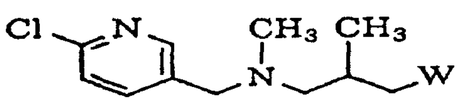

( I )

wherein W is a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group, 3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group, 2,3,5,6-tetrafluoro-4-trifluoromethylphenyl group, 4-trifluoromethoxyphenyl group, 4-difluoromethoxyphenyl group, 4-(1,1,2,3,3,3-hexafluoropropoxy) phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group, 3,5-difluoro-4-trifluoromethoxyphenyl group, 3-chloro-5-fluorophenyl group, 3,5-dichloro-4-fluorophenyl group, pentafluorophenyl group, 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl group and 2,2,4,4-tetrafluoro-4H-1,3- benzodioxin-6-yl group. In 6-chloro-3-pyridylmethylpropylamine derivatives represented by the foregoing formula (I), the second position carbon in the propyl group is an asymmetric carbon, and thus there can be optical isomers regardless of chirality at other substituents. However, the optical isomers may be used singly and mixedly in the present invention.

More concretely, 6-chloro-3-pyridylmethylpropylamine derivatives of the present invention are the compounds I-1 to 27 that include the groups represented by W in the aforementioned formula (I) that are described in the Table 1 to 4 and the Fig. 1A to 1D, specifically, a fluoromethylphenyl group described in Table 1 and Fig. 1A, a fluoroalkoxyphenyl group described in Table 2 and Fig. 1B, a halogen-substituted phenyl group described in Table 3 and Fig. 1C and benzodioxol group and benzodioxin group described in Table 4 and Fig. 1D. A dot '•' in the Fig. 1A to 1D represents the third position carbon in the propyl group that is bonded to W in the foregoing formula (I).

[Table 1]

| Compound No. | W |
|---|---|
| I-1 | 3,5-bistrifluoromethylphenyl |

[Table 1] (continued)

| Compound No. | W |
|---|---|
| I-2 | 2,4-bistrifluoromethylphenyl |
| I-3 | 2,5-bistrifluoromethylphenyl |
| I-4 | 3-difluoromethyl-5-trifluoromethylphenyl |
| I-5 | 3-chloro-5-trifluoromethylphenyl |
| I-6 | 3-fluoro-5-trifluoromethylphenyl |
| I-7 | 2-fluoro-3-trifluoromethylphenyl |
| I-8 | 2-fluoro-4-trifluoromethylphenyl |
| I-9 | 3-fluoro-4-trifluoromethylphenyl |
| I-10 | 2,3-difluoro-4-trifluoromethylphenyl |
| I-11 | 2,3,5,6-tetrafluoro-4-trifluoromethylphenyl |

[Table 2]

| Compound No. | W |
|---|---|
| I-12 | 4-trifluoromethoxyphenyl |
| I-13 | 4-difluoromethoxyphenyl |
| I-14 | 4-(1,1,2,3,3,3-hexafluoropropoxy) phenyl |
| I-15 | 4-chloro-3-trifluoromethoxyphenyl |
| I-16 | 4-fluoro-3-trifluoromethoxyphenyl |
| I-17 | 3-fluoro-4-trifluoromethoxyphenyl |
| I-18 | 3-chloro-4-trifluoromethoxyphenyl |
| I-19 | 3-chloro-4-bromodifluoromethoxyphenyl |
| I-20 | 3,5-difluoro-4-trifluoromethoxyphenyl |

[Table 3]

| Compound No. | W |
|---|---|
| 1-21 | 3-chloro-5-fluorophenyl |
| 1-22 | 3,5-dichloro-4-fluorophenyl |
| 1-23 | Pentafluorophenyl |

[Table 4]

| Compound No. | W |
|---|---|
| 1-24 | 2,2-difluoro-1,3-benzodioxol-5-yl |
| 1-25 | 2,2-dichloro-1,3-benzodioxol-5-yl |
| 1-26 | 2,2,3,3-tetrafluoro-2,3-dihydro-1,4-benzodioxin-6-yl |
| 1-27 | 2,2,4,4-tetrafluoro-4H-1,3-benzodioxin-6-yl |

(The preparation method of propylamine derivatives)

6-chloro-3-pyridylmethylpropylamine derivatives of the present invention are explained below on the basis of

their preparation method.

6-chloro-3-pyridylmethylpropylamine derivatives of the present invention are preferably obtained by the following reaction formula (A):

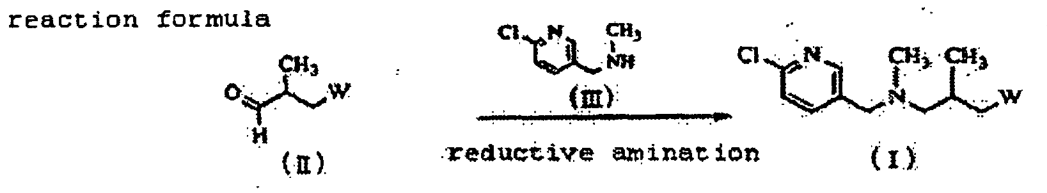


wherein W has the same definition as that described above. More specifically, 6-chloro-3-pyridylmethylpropylamine derivatives (I) are obtained by a reductive amination in the presence of a reducing agent using 3-phenylpropionaldehyde derivative (II) that has a specific structure and 6-chloro-3-pyridylmethylamine derivative (III) where the hydrogen atom at sixth position carbon of the heterocyclic group is substituted with a chlorine atom. Further, 6-chloro-3-pyridylmethylpropylamine derivatives are preferably obtained by the following reaction formula (B) :

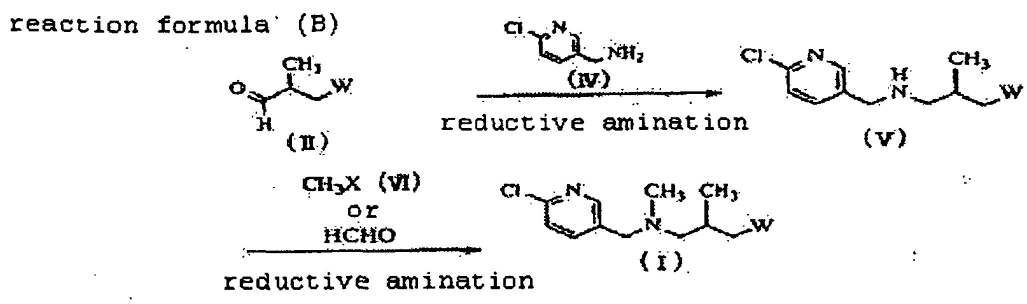


wherein W has the same definition as that described above, and X represents a leaving group. More specifically, 6-chloro-3-pyridylmethylpropylamine derivatives (I) are obtained by a reductive amination wherein 3-phenylpropionaldehyde derivative (II) and 6-chloro-3-pyridylmethylamine derivative (IV) are used to form 3-phenylpropylamine derivative (V), and then react the foregoing 3-phenylpropylamine derivative (V) with at least one of the methylating agents selected from the group consisting of the compound (VI) and formaldehyde.

Herein, compound (II) can be synthesized referring to the method described in the following literature: Tetrahedron Letters 8, 597 (1976),
and applying the reaction formula (C) mentioned below: reaction formula (c)

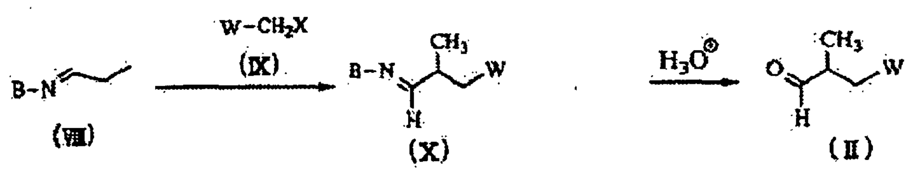


wherein W has the same definition as that defined above, and B represents a C3-10 alkyl group or a C3-10 cycloalkyl group, and X represents a leaving group. specifically, 3-phenylpropionaldehyde derivatives (II) are synthesized by acid hydrolysis of compound (X) that is obtained by reacting imine derivatives (VIII) and benzyl compound (IX) in the presence of a base; or 3-phenylpropionaldehyde derivatives (II) may be synthesized by the following reaction formula (D):

reaction formula (D)

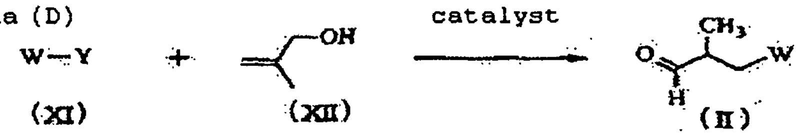


(XI) (XII) (II)

wherein W has the same definition as that defined above, and Y represents a bromine atom or an iodine atom. More specifically, they are synthesized by reacting halogenated benzene derivatives (XI) and methacrylalcohol (XII) with palladium catalyst.

Some of pyridylmethylamine derivatives (III) and (IV), imine derivatives (VIII), benzyl compounds (IX), and halogenated benzene derivatives (XI) that are used in the aforementioned methods are commercially available, or may be synthesized by the methods known in the prior art described in Japanese Unexamined Patent Publication No. HEI 2-171; Japanese Unexamined Patent Publication No. HEI 3-223252; Tetrahedron Letters 8, 597 (1976) ; ibid., 17, 1379 (1976).

Methylating agents (VI) include methyliodide, methylbromide, methylchloride, dimethylsulphate, p-toluenesulfonicacidmethylester, and the like.

Imine derivatives (VIII) include propylidene-t-butylamine, propylidenecyclohexylamine, and the like;

Benzyl compounds (IX) include 3,5-bistrifluoromethylbenzyl bromide, pentafluorobenzyl bromide, 2,4- bistrifluoromethylbenzyl bromide, 2,3,5,6- tetrafluoro-4-trifluoromethylbenzyl bromide, 2,2-difluoro-1,3-benzodioxol-5-ylmethyl bromide, 3-difluoromethyl-5-trifluoromethylbenzyl bromide, 2-fluoro-3-trifluoromethylbenzyl bromide, 2,5-bistrifluoromethylbenzyl bromide, 3-fluoro-5-trifluoromethylbenzyl bromide, 4-trifluoromethoxybenzyl bromide, 4- trifluoromethoxybenzyl chloride, 2,3-difluoro-4-trifluoromethylbenzyl bromide, 3-trifluoromethyl-4-chlorobenzyl bromide, 3-chloro-5-fluorobenzyl bromide, 2,2-dichloro-1,3-benzodioxol-5-ylmethyl bromide, 6-bromomethyl-2,2,3,3-tetrafluoro-2,3-dihydro-1,4-benzo dioxin, 3-chloro-5-trifluoromethylbenzyl bromide, 3-trifluoromethoxy-4-fluorobenzyl bromide, 3-fluoro-4-trifluoromethoxybenzyl bromide, 3,5-difluoro-4-trifluoromethoxybenzyl bromide, 2-fluoro-4-trifluoromethylbenzyl bromide, 3-fluoro-4-trifluoromethylbenzyl bromide, 3,5-dichloro-4-fluorobenzyl bromide, 4-(1,1,2,3,3,3-hexafluoropropoxy) benzyl bromide, 2,2,4,4-tetrafluoro-4H-1,3-benzodioxin-6-ylmethyl bromide, and the like.

Benzyl compounds (IX) having a leaving group X include halogenide, sulfuric ester, non-substituted or substituted sulfonic acid ester, and the like. Apreferable leaving group X is a halogen atom such as chlorine, bromine and iodine or a p-toluene sulfonyloxy group.

Halogenated benzene derivatives (XI) include 3,5-bistrifluoromethylbromobenzene, pentafluorobromobenzene, 2,4-bistrifluoromethylbromobenzene, 2,3,5,6-tetrafluoro-4-trifluoromethylbromobenzene, 3-difluoromethyl-5-trifluoromethylbromobenzene, 2-fluoro-3-trifluoromethylbromobenzene, 2,5-bistrifluoromethylbromobenzene, 3-fluoro-5-trifluoromethylbromobenzene, 4- trifluoromethoxybromobenzene, 4- trifluoromethoxyiodobenzene, 2,3-difluoro-4-trifluoromethylbromobenzene, 3-trifluoromethoxy-4-chlorobromobenzene, 3-chloro-5-fluorobromobenzene, 3-chloro-5-trifluoromethylbromobenzene, 3-trifluoromethoxy-4-fluorobromobenzene, 3-fluoro-4-trifluoromethoxybromobenzene, 3,5-difluoro-4-trifluoromethoxybromobenzene, 2-fluoro-4-trifluoromethylbromobenzene, 3-fluoro-4-trifluoromethylbromobenzene, 3,5,-dichloro-4-fluorobromobenzene, 4-(1,1,2,3,3,3-hexafluoropropoxy) bromobenzene, 4-(difluoromethoxy) bromobenzene, 3-chloro-4-(bromodifluoromethoxy) bromobenzene, 3-chloro-4-(trifluoromethoxy) bromobenzene, 3,4-difluoro-5-trifluoromethylbromobenzene, 5-bromo-2,2-difluoro-1,3-benzodioxol, 5-bromo-2,2-dichloro-1,3-benzodioxol, 6-bromo-2,2,3,3-tetrafluoro-2,3-dihydro-1,4-benzodioxin, 6-bromo-2,2,4,4-tetrafluoro-4H-1,3-benzodioxin, and the like.

(Reductive amination)

As mentioned above, in the preparation method of 6-chloro-3-pyridylmethylpropylamine derivatives of the present invention, a reductive amination is employed during the synthesis process of the targeted compounds or the intermediates for them. Such reductive amination may be performed by the methods described in the literature including
J. Am. Chem. Soc., 93, 2897 (1971);
Synthesis, 135 (1975);
Org. React., 4, 174 (1948);
J. Org. Chem., 61, 3849 (1996); and
Tetrahedron Letters, 31, 5595 (1990).

The preferred reducers for the aforementioned reductive amination are complex hydrogen compounds such as triacetoxy sodium borohydrideand cyanosodium borohydride. In addition, combinations of hydrogen gas and palladium/hydrogenising agents such as charcoal, Raney nickel and formic acid may be also preferably used.

Further, the aforementioned reductive amination may be performed in solvent or without solvent. Solvents

that are used for the reaction in solvent include alcohols such as methanol and ethanol; ethers such as tetrahydrofuran and dioxane; halogenated hydrocarbons such as 1,2-dichloroethane; water; and acetonitrile. These solvents may be used singly and may be used in mixtures that contain at least one of them.

Furthermore, there is no particular limitation on the reaction condition in the aforementioned reductive amination. However, the amount of the aforementioned reducer against that of 3-phenylpropionaldehyde derivative (II) or formaldehyde is preferably in the range of 1.0 to 20.0 by mole ratio and more preferably in the range of 1.0 to 3.0. If the mole ratio of the reducer is less than 1.0, the reaction yield is likely to decrease, and if it is greater than 20.0, the amount of reducer tends to be excessive in the reaction. Besides, the amount of 6-chloro-3-pyridylmethylamine derivative (III), (IV) and formaldehyde against that of compound (II) or 3-phenylpropylamine derivative (V) may be each preferably in the range of 1. 0 to 20.0 by mole ratio and more preferably in the range of 1.0 to 3.0. If the mole ratio of 6-chloro-3-pyridylmethylamine derivative (III), (IV) and formaldehyde is less than 1.0, the reaction yield is likely to decrease, and if it is greater than 20.0, the amount of reducer tends to be excessive in the reaction. Although the reductive amination is normally performed between the temperature of the melting point and the boiling point of the used solvent, it is desirable that the aforementioned reductive amination be performed in the temperature range of at least 20 °C and no more than 50 °C. If the reaction temperature is outside the foregoing range, the reaction yield is likely to decrease.

(Alkylation)

Alkylation involved in the present invention is explained below, which is the process that gives propylamine derivatives (I) from 3-phenylpropylamine derivatives (V) and methylating agents (VI) in the aforementioned reaction formula (B) and the process that gives compound (X) from imine derivatives (VIII) and benzyl compounds (IX) in the aforementioned reaction formula (C).

The reaction condition in a general alkylation may be applied for the alkylation involved in the present invention. This reaction may be performed in solvent or without solvent.

Solvents that are used for alkylation in solvent include hydrocarbons such as benzene, toluene, xylene and hexane; halogenated hydrocarbons such as methylene chloride, chloroform and carbon tetrachloride; ethers such as diethylether, diisopropylether, tetrahydrofuran and dioxane; ketones such as acetone and methylethylketone; and others such as acetonitrile, N,N-dimethylformamide, 1-methyl-2-pyrrolidinone and dimethylsulfoxide.

It is desirable that the aforementioned alkylation be performed in the presence of a base. Alkylation is likely to be stimulated in the presence of a base. The usable bases include inorganic bases such as sodium carbonate, potassium carbonate, sodium hydrogencarbonate, sodium hydroxide and potassium hydroxide; alkoxides of alkaline metals such as sodium methoxide, sodium ethoxide and potassiumt-butoxide; alkaline metal hydrogen compounds such as sodium hydride and potassium hydride; organic metal compounds of alkaline metals such as lithium diisopropylamideand n-butyllithium; and organic tertiary amines such as triethylamine, pyridine, N,N-dimethylaniline, DBU (1,8-diazabicyclo[5.4.0] undese-7-ene). Inorganic bases such as sodium carbonate and potassium carbonate are particularly preferable to use in the aforementioned reaction formula (B) to stimulate the alkylation and facilitate the handling of the reagents. In addition, the organic metal compounds of alkaline metals such as lithium diisopropylamide and n-butyllithium are particularly preferable to use in the aforementioned reaction formula (C) to stimulate the alkylation. Further, the amount of the foregoing base against that of 3-phenylpropylamine derivative (V) or imine derivative (VIII) is preferably in the range of 1.0 to 10.0 by mole ratio, and more preferably in the range of 1.0 to 2.0. If the mole ratio of base against 3-phenylpropylamine derivative (V) or imine derivative (VIII) is less than 1.0, the reaction yield is likely to decrease. If the ratio is greater than 10.0, the amount of base tends to be excessive .

Furthermore, the amount of methylating agent (VI) and benzyl compound (IX) against that of compound (V) and (VIII) may be each preferably in the range of 1.0 to 20.0 and more preferably in the range of 1.0 to 4.0.

Although the foregoing alkylation is normally performed between the temperature of the melting point and the boiling point of the used solvent, it is preferable that the alkylation in the aforementioned reaction formula (B) is performed in the range of 20 °C to 100 °C. If the reaction temperature of alkylation in the reaction formula (B) is outside the aforementioned range, the reaction yield is likely to decrease. On the other hand, the alkylation in the reaction formula (C) is preferably performed in the range of -78 °C to 20 °C. If the reaction temperature of alkylation in the reaction formula (C) is outside the aforementioned range, the reaction yield is likely to decrease.

(Arylation)

The arylation in the foregoing reaction formula (D), namely, the synthesis reaction of 3-phenylpropionaldehyde derivatives (II) from substituted halogenated benzene derivatives (XI) and methacryl alcohol (XII) is preferably performed in the presence of palladium catalyst. More specifically, the methods described in

J. Org. Chem., 41, 273 (1976) ; ibid., 41, 1206 (1976), ibid., 47, 2117 (1982)

may be applied to the aforementioned arylation. The used Palladium catalysts include palladium chloride (II), palladium acetate (II), and the like.

(Purification processing)

After completion of the reaction represented by the reaction formula (A) or (B) described above, the obtained reaction mixture is purified to give the targeted compound (I), wherein the method known in the prior art may be applied for the aforementioned purification processing. More specifically, the reaction mixture resulting from the reaction described above is poured into ice water followed by extraction with an organic solvent such as ethylacetate, chloroform, methylene chloride and benzene to separate the organic layer. Thereafter, this organic layer is washed with water and dried. After the solvent is removed under the reduced pressure, the resulting residue is purified by silica gel chromatography to give the target compound (I).

6-Chloro-3-pyridylmethylpropylamine derivatives (I) obtained by the aforementioned method contain an asymmetric carbon at the second position carbon of the propyl group. Therefore, there may be optical isomers regardless of chirality at other substituents. The compound (I) of the present invention includes all single isomers and mixtures of each isomer at any mixing ratio.

(Acid addition salts)

The foregoing 6-chloro-3-pyridylmethylpropylamine derivatives (I) may easily form acid addition salts and thus may be used in a form of either inorganic acid salt or organic acid salt. Acids that form acid addition salts include inorganic acids such as hydrochloric acid, hydrobromic acid, hydriodic acid, nitric acid, sulfuric acid and phosphoric acid; organic acids such as formate, acetate, lactate, p-toluenesulfonic acid, maleic acid, succinic acid, fumaric acid, tartaric acid, citric acid, salicylic acid, dodecylbenzene sulfonic acid, methane sulfonic acid, camphorsulfonic acid, saccharine, oxalic acid, palmitic acid, stearic acid and benzoic acid.

(Fungicidal action against plant pathogens)

6-Chloro-3-pyridylmethylpropylamine derivatives (I) of the present invention have a control effect on plant diseases such as Pyricularia oryzae, Cochliobolus miyabeanus, Xanthomonas oryzae, Rhizoctonia solani, Helminthosporiumsigmoideun, Gibberellafujikuroi, Pythium aphanidermatum, Podosphaera leucotricha, Venturia inaequalis, Monilinia mali, Alternaria alternata, Valsa mali, Alternaria kikuchiana, Phyllactinia pyri, Gymnosporangium asiaticum, Venturia nashicola, Uncinula necator, Plasmopara viticola, Glomerella cingulata, Erysiphe graminis f. sp hordei, Puccinia graminis, Puccinia striiformis,Pyrenophora graminea,Rhynchosporiumsecalis, Erysiphe graminis f. sp tritici, Puccinia recondita, Pucciniastriiformis, Pseudocercosporellaherpotrichoides, Microdochium nivale, Leptosphaeria nodorum, Septoria tritici, Sphaerotheca fuliginea, Colletotrichum lagenarium, Pseudoperonospora cubensis,Phytophthora capsici,Erysiphe cichoracearum, Alternaria solani, Erysiphe cichoracearum, Sphaerotheca humuli, Erysiphe cichoracearum, Cercospora beticola, Ustillaga maydis, Monilinia fructicola, Botrytis cinerea and Sclerotinia sclerotiorum. The forgoping 6-chloro-3-pyridylmethylpropylamine derivatives (I) also have a therapeutic effect as well as a preventive control effect on some of the plant diseases described above.

(Effective ingredient for fungicide)

When 6-chloro-3-pyridylmethylpropylamine derivatives (I) of the present invention are used as an effective ingredient of fungicides, they can be used alone for fungicides (e.g., agrochemicals). However, they are normally used in various forms such as dusting powder, wettable powder, granule and emulsifiable concentrate after having been formulated together with formulating adjuvants to be used, where necessary.

Herein, the content of the compound (I) of the present invention in the preparation of fungicides preferably ranges from 0.1 to 95% by weight, more preferably from 0.5 to 90% by weight, and most preferably from 2 to 70% by weight with respect to the total amount of the fungicide (100% by weight), which contains the compound (I) of the present invention. If the content of the compound (I) is less than 0.1% by weight, the fungicide does not show enough control effects against plant diseases. If the content of the compound (I) exceeds 95% by weight, the fungicide leads to insufficient control effects due to the shortage of the formulating adjuvants. When the fungicide comprises more than one kind of the compound (I) , the content of the foregoing compound (I) means the total weight of the compound (I).

The formulating adjuvants include such as solid carrier, liquid diluent and surfactant. Talc, kaolin, bentonite, diatomaceous earth, white carbon, clay and the like are suitably used for solid carrier. Water, xylene, toluene, chlorobenzene, cyclohexane, cyclohexanone, dimethylsulfoxide, dimethylformamide, alcohol and the like are suitably used for liquid diluents. Preferably, surfactants are used depending on their effects, where appropriate. Examples of emulsifying agents are polyoxyethylenealkylallylether, polyoxyethylene sorbitan monolaurate and the like. Examples of dispersing agents are lignin sulfonate, dibutyl naphthalenesulfonate and the like. Examples of wetting agents are alkylsulfonate, alkylphenylsulfonate, alkylsulfate and the like.

Some of the aforementioned fungicides can be used as they are, and others may be used after having been diluted to their predetermined concentrations with diluents such as water. When the fungicides are diluted to use, the concentration of the compound (I) of the present invention in the diluted liquid preferably ranges from 0.001 to 1.0% by weight. If the concentration of the compound (I) is less than 0.001% by weight, the fungicide is virtually inactive for plant diseases. If the concentration of the compound (I) is more than 1.0% by weight, the amount of the compound (I) results in an excess dispersal amount. The use level of the compound of the present invention preferably ranges from 20 to 5000 g, and more preferably from 50 to 1000 g, per hectare (ha) of the farming or horticultural land such as a field, rice paddy, orchard or greenhouse. If the amount of use is less than 20g, the fungicide is virtually inactive for plant diseases. If the amount is more than 5000g, it results in an excessive use of the fungicide. Because these concentrations and amounts for use of the compound (I) in the fungicide will vary according to the preparation form, use timing, use method, location of application, the crop objects and other factors, they may be increased or reduced from the aforementioned ranges where necessary. Further, the compounds of the present invention may be used in combination with other effective ingredients such as fungicide, insecticides, acaricide and herbicides, which are not included in the present invention, if necessary.

## Examples

The present invention will now be explained in detail by referring to preparation examples, formulation examples and test examples. However, the invention is not limited to thefollowing preparationexamples,formulation examples and test examples as long as it does not depart from the gist thereof.

Preparation Example 1

According to the procedure shown below, 6-chloro-N-[3-(3-fluoro-4-trifluoromethylphenyl)-2-meth ylpropyl] -N-methyl-3-pyridylmethylamine [I-9] was prepared. I-9 denotes the compound number listed in Table 1 . In the same manner, the compound numbers listed in Table 1 to 4 are denoted.

3-(3-fluoro-4-trifluoromethylphenyl)-2-methylprop anal [In the formula (II), W represents 3-fluoro-4-trifluoromethylphenyl group] was prepared using the procedure described by the reaction formula (C). Namely, 5.4 ml (8.1 mmol) of a 1.5M solution of lithium diisopropylamide (LDA) in cyclohexane was cooled to 0°C, and 4 ml of anhydrous THF and 1.4 g (10.0 mmol) of propylidene cyclohexylamine were added to the solution and stirred for 30 minutes under nitrogen atmosphere. The resultant solution was cooled to -70°C. Another solution which was provided by dissolving 1.0 g (3.9 mmol) of 3-fluoro-4-trifluoromethylbenzylbromide in 2 ml of anhydrous THF was dropped into the solution and stirred at - 70°C for 1 hour.

Subsequently, the reaction temperature of the solution returned to 0°C, and 16 ml of a 2N hydrochloric acid was added to the solution and stirred at ambient temperature for 1 hour. The resultant solution was neutralized with 2N sodium hydroxide aqueous solution and extracted with benzene. The separated benzene phase was washed with water and saturated brine, and then dried over sodium sulfate anhydride. After solvent removal by distillation under a reduced pressure, 1.5 g of an oily substance was obtained. The oily substance was purified by using silica gel column (Merck Silica gel 60, 230-400 mesh, Merck Co., eluent composition: Hexane /AcOEt=15/1). This gave 480 mg of 3-(3-fluoro-4-trifluoromethylphenyl)-2-methylpropanal as a yellowish oily substance. The yield was 52.6%.

The molecular structure of the obtained compound was confirmed by using 1H-NMR. The results are shown below.

1H-NMR ($CDCl_3$, δ, ppm) : 1.11 (d, 3H, J=6Hz) , 2.33-3.27 (m, 3H), 6.80-7.65 (m, 3H), 9.60 (d, 1H, J=1Hz).

Next, according to the procedure represented by the foregoing reaction formula (A), 480 mg (2.0 mmol) of obtained 3-(3-fluoro-4-trifluoromethyphenyl)-2-methylpropanal and 390 mg (2.5 mmol) of 6-chloro-N-methyl-3-pyridylmethylamine were dissolved in 7 ml of 1,2-dichloroethane. After adding 530 mg (2.5 mmol) of sodium triacetoxyboronhydride, the solution was stirred at ambient temperature for 1 hour.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 810 mg of an oily substance was obtained. The oily substance was purifiedby using silica gel column (Merck Silica gel 60, 230-400 mesh, Merck Co., eluent composision:Hexane /AcOEt=5/1). This gave 700 mg of the desired compound [I-9] as a clear oily substance. The yield was 91.2%.

Preparation Example 2

According to the procedure shown below, 6-chloro-N-methyl-N-[3-(4-trifluoromethoxyphenyl)-2-met hylpropyl]-3-pyridylmethylamine [I-12] was prepared.

2-methyl-3-(4-trifluoromethoxyphenyl)propanal [in the formula (II), W represents 4-trifluoromethoxyphenyl group] was prepared using the procedure described by the reaction formula (D). 16 mg (0.09mmol) of palladium chloride (II) and 46 mg (0.18 mmol) of triphenylphosphine were dissolved in 4 ml of N,N-dimethylformamide at 120°C under nitrogen atmosphere, and allowed to cool to 50°C. The solution was admixed with 2.0 g (8.3 mmol) of 4-trifluoromethoxybromobenzene, 900 mg (12.5 mmol) of methallyl alcohol (equivalent of 2-methyl-2-propene-1-ol) and 840 mg (10 mmol) of sodium hydrogencarbonate and stirred at 125°C for 1 hour.

After standing to cool, the resultant solution was admixed with water and extracted with benzene. The separated benzene phase was washed with water and saturated brine successively and dried over sodium sulfate anhydride. After solvent removal by distillation under a reduced pressure, 1.7 g of an oily substance was obtained. The oily substance was purified by using silica gel column (Merck Silica gel 60, 230-400 mesh, Merck Co., eluent composition: Hexane/AcOEt=10/1). This gave 1.27 g of 2-methyl-3-(4-trifluoromethoxyphenyl)propanal as an oily substance. The yield was 66.0%.

The molecular structure of the obtained compound was confirmed by using 1H-NMR. The results are shown below. 1H-NMR ($CDCl_3$, δ, ppm) : 1.11 (d, 3H, J=7Hz) , 2. 56-2. 68 (m, 2H), 3.10 (dd, 1H, J=5, 13Hz), 7.14 (d, 2H, J=9Hz), 7.20 (d, 2H, J=9Hz), 9.72 (d, 1H, J=1.5Hz).

Next, according to the procedure represented by the aforementioned reaction formula (A), 1.27 g (5.5 mmol) of 2-methyl-3-(4-trifluoromethoxyphenyl)propanal and 1.0 g (6.4 mmol) of 6-chloro-N-methyl-3-pyridylmethylamine were dissolved in 15 ml of 1, 2-dichloroethane. After adding 1 . 4 g (6.6 mmol) of sodium triacetoxyboronhydride, the solution was stirred at ambient temperature for 2 hours.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 2.1 g of an oily substance was obtained. The oily substance was purified by using silica gel column (Merck Silica gel 60, 230-400 mesh, Merck Co., eluent composition: Hexane /AcOEt=5/1). This gave 1.78 g of the desired compound [I-12] as a clear oily substance. The yield was 86.9%.

Preparation Example 3

According to the procedure shown below, 6-chloro-N-[3-(3-chloro-5-fluorophenyl)-2-methylpropyl] -N-methyl-3-pyridylmethylamine [I-21] was prepared.

3-(3-chloro-5-fluorophenyl)-2-methylpropanal [in the formula (II), W represents 3-chloro-5-fluorophenyl group] was prepared using the procedure described by the reaction formula (D). 8 mg (0.045mmol) of palladium chloride (II) and 24 mg (0.09 mmol) of triphenylphosphine were dissolved in 2 ml of hexamethylphosphoramide at 120°C under nitrogen atmosphere, and allowed to cool to 50°C. The solution was admixed with 1.0 g (4.8 mmol) of 3-chloro-5-fluorobromobenzene, 520 mg (7.2 mmol) of methallyl alcohol and 480 mg (5.7 mmol) of sodium hydrogencarbonate, and stirred at 130°C for 3 hours.

After standing to cool, the resultant solution was admixed with water and extracted with benzene. The separated benzene phase was washed with water and saturated brine successively, and dried over sodium sulfate anhydride. After solvent removal by distillation under a reduced pressure, 860 mg of an oily substance was obtained. The oily substance was purified by using silica gel column (Merck Silica gel 60, 230-400 mesh, Merck Co., eluent composition: Hexane /AcOEt=15/1). This gave 490 mg of 3-(3-chloro-5-fluorophenyl)-2-methylpropanal as an oily substance. The yield was 50.9%.

The molecular structure of the compound obtained was confirmed by using 1H-NMR. The results are shown below. 1H-NMR ($CDCl_3$, δ, ppm) : 1.10 (d, 3H, J=6Hz), 2.27-3.22 (m, 3H), 6.55-7.03 (m, 3H), 9.62 (d, 1H, J=1Hz).

Next, according to the procedure represented by the aforementioned reaction formula (A) , 300 mg (1.5 mmol) of 3-(3-chloro-5-fluorophenyl)-2-methylpropanal and 280 mg (1.8 mmol) of 6-chloro-N-methyl-3-pyridylmethylamine were dissolved in 4 ml of 1,2-dichloroethane. After adding 380 mg (1.8mmol) of sodium triacetoxyboronhydride, the solution was stirred at ambient temperature for 2 hours.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 470 mg of an oily substance was obtained. The oilysubstancewaspurifiedbyusing silica gel column (Merck Silica gel 60, 230-400 mesh, Merck Co., eluent composition: Hexane/AcOEt=5/1). This gave 500 mg of the desired compound [I-21] was obtained as a clear oily substance. The yield was 97.8%.

Preparation Example 4

According to the procedure shown below, 6-chloro-N-[3-(3-fluoro-4-trifluoromethoxyphenyl)-2-met hylpropyl] -N-methyl-3-pyridylmethylamine [I-17] was prepared.

3-(3-fluoro-4-trifluoromethoxyphenyl)-2-methylpro panal [in the formula (II), W represents 3-fluoro-4-trifluoromethoxyphenyl group] was prepared using the method described by the reaction formula (D) . 7 mg (0.04 mmol) of palladium chloride (II) and 20 mg (0.076 mmol) of triphenylphosphine were dissolved in 2 ml of N,N-dimethylformamide at 120°C under nitrogen atmosphere, and allowed to cool to 50°C. The solution was admixed with 1.0 g (3.9 mmol) of 3-fluoro-4-trifluoromethoxybromobenzene, 420mg (5.8mmol) of methallyl alcohol and 390 mg (4.6 mmol) of sodium hydrogencarbonate, and was stirred at 130°C for 3 hours.

After standing to cool, the resultant solution was admixed with water and extracted with benzene. The separated benzene phase was washed with water and saturated brine successively, and dried over sodium sulfate anhydride. After solvent removal by distillation under a reduced pressure, 870 mg of an oily substance was obtained. The oily substance was purified by using silica gel column (Merck Silica gel 60, 230-400 mesh, Merck Co., eluent composition: Hexane/AcOEt=15/1). This gave 500 mg of 3-(3-fluoro-4-trifluoromethoxyphenyl)-2-methylpropanal as an oily substance. The yield was 51.3%.

The molecular structure of the obtained compound was confirmed by using 1H-NMR. The results are shown below; 1H-NMR ($CDCl_3$, $\delta$, ppm) : 1.10 (d, 3H, J=6Hz) , 2.27-3.23 (m, 3H), 6.73-7.45 (m, 3H), 9.68 (d, 1H, J=1Hz).

Next, according to the procedure represented by the aforementioned reaction formula (A), 380 mg (1.5 mmol) of the obtained 3-(3-fluoro-4-trifluoromethoxyphenyl)-2-methylpropanal and 290 mg (1.9 mmol) 6-chloro-N-methyl-3-pyridylmethylamine were dissolved in 5 ml of 1,2-dichloroethane. After adding 400 mg (1.9 mmol) of sodium triacetoxyboronhydride, the solution was stirred at ambient temperature for 2 hours.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 590 mg of an oily substance was obtained. The oily substance was purified by using silica gel column (Merck Silica gel 60, 230-400 mesh, Merck Co., eluent composition: Hexane/AcOEt=5/1). This gave 500 mg of the desired compound [I-17] as a clear oily substance. The yield was 85.3%.

Preparation Example 5

According to the procedure shown below, 6-chloro-N-[3-(3,5-difluoro-4-trifluoromethoxyphenyl)-2 -methylpropyl]-N-methyl-3-pyridylmethylamine [I-20] was prepared.

3-(3,5-difluoro-4-trifluoromethoxyphenyl)-2-methy lpropanal [in the formula (II), W represents 3,5-difluoro-4-trifluoromethoxyphenyl group] was prepared using the procedure described by the reaction formula (D). 7 mg (0.04 mmol) of palladium chloride (II) and.20 mg (0.076 mmol) of triphenylphosphine were dissolved in 2 ml of dimethylformamide at 120°C under nitrogen atmosphere, and allowed to cool to 50°C. The solution was admixed with 1.0 g (3.6 mmol) of 3,5-difluoro-4-trifluoromethoxybromobenzene, 390 mg (5.4 mmol) of methallyl alcohol and 370 mg (4.4 mmol) of sodium hydrogencarbonate, and stirred at 130°C for 3 hours.

After standing to cool, the resultant solution was admixed with water and extracted with benzene. The separated benzene phase was washed with water and saturated brine successively, and dried over sodium sulfate anhydride. After solvent removal by distillation under a reduced pressure, 900 mg of an oily substance was obtained. The oily substance was purified by using silica gel column (Merck Silica gel 60, 230-400 mesh, Merck Co., eluent composition: Hexane/AcOEt=10/1). This gave 300 mg of 3-(3,5-difluoro-4-trifluoromethoxyphenyl)-2-methylpropa nal as an oily substance. The yield was 31.1%.

The molecular structure of the obtained compound was confirmed by using 1H-NMR. The results are shown below.

1H-NMR ($CDCl_3$, $\delta$, ppm) : 1.10 (d, 3H, J=6Hz) , 2.33-3.17 (m, 3H), 6.50-7.03 (m, 2H), 9.63 (d, 1H, J=1Hz).

Next, according to the procedure represented by aforementioned reaction formula (A) , 240 mg (0.90 mmol) of 3-(3,5-difluoro-4-trifluoromethoxyphenyl)-2-methylpropa nal and 6-chloro-N-methyl-3-pyridylmethylamine (170 mg, 1.08 mmol) were dissolved in 4 ml of a 1, 2-dichloroethane. After adding 240 mg (1.13 mmol) of sodium triacetoxyboronhydride, the solution was stirred at ambient temperature for 2 hours.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 450 mg of an oily substance was obtained. The oily substance was purifiedby using silica gel column (Merck Silica gel 60, 230-400 mesh, Merck Co., eluent composition: Hexane/AcOEt=5/1). This gave 300 mg of the desired compound [I-20] as a clear oily substance. The yield was 81.6%

Preparation Example 6

According to the procedure presented by the aforementioned reaction formula (B), 6-chloro-N-methyl-[3-(4-trifluoromethoxyphenyl)-2-methy lpropyl]-3-pyridylmethylamine [I-12] was prepared using the following steps.

1.87 g (8.1 mmol) of 3-(4-trifluoromethoxyphenyl)-2-methylpropanal obtained by the procedure analogous to Preparation Example 2 and 1.15 g (8.1 mmol) of 6-chloro-3-pyridylmethylamine were dissolved in 18 ml of 1, 2-dichloroethane. After adding 2.1 g (9.9 mmol) of sodium triacetoxyboronhydride, the solution was stirred at ambient temperature for 1 hour.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 2.4 g of an oily substance was obtained. The oily substance was purified by using silica gel column (Merck Silica gel 60, 230-400 mesh, Merck Co. , eluent composition: Hexane/AcOEt=1/1, AcOEt). This gave 2.2 g of 6-chloro-N-[3-(4-trifluoromethoxyphenyl)-2-methylpropyl ]-3-pyridylmethylamine as a clear oily substance. The yield was 75.8%

The molecular structure of the obtained compound was confirmed by using 1H-NMR. The results are shown below. 1H-NMR ($CDCl_3$, δ, ppm) : 0.85 (d, 3H, J=6.4Hz) , 1.28 (s, 1H) , 1 . 53-2. 90 (m, 5H) 3 . 65 (s, 2H) , 7. 03 (s, 4H) , 7.13 (d, 1H, J=8Hz), 7.53 (dd, 1H, J=2, 8 Hz), 8.22 (d, 1H, J=2.0Hz).

Next, 500 mg (1.4 mmol) of the obtained 6-chloro-N-[3-(4-trifluoromethoxyphenyl)-2-methylpropyl ]-3-pyridylamine and 200 mg (1.4 mmol) of methyl iodide were dissolved in 2 ml of N,N-dimethylformamide. The solution was admixed with 290 mg (2.1 mmol) of potassium carbonate and stirred at 35°C for 1 hour.

Thereafter, the resultant solution was poured into water and the mixture was extracted with ethyl acetate. The separated ethyl acetate phase was washed with water and saturated brine, and dried over sodium sulfate anhydride. After solvent removal by distillation under a reduced pressure, 500 mg of an oily substance was obtained. The oily substance was purifiedby using silica gel column (Merck Silica gel 60, 230-400 mesh, Merck Co., eluent composition: Hexane/AcOEt=5/1). This gave 400 mg of the desired compound [I-12] as a clear oily substance. The yield was 76.7%.

Preparation Example 7

Accordingtotheprocedurepresentedbytheaforementionedreactionformula(B),6-chloro-N-methyl-N-[3-(4-trifluoromethoxyphenyl)-2-met hylpropyl]-3-pyridylmethylamine [I-12] was prepared using the following steps.

500 mg (1.4 mmol) of 6-chloro-N-(3-(4-trifluoromethoxyphenyl)-2-methylpropyl ]-3-pyridylmethylamine prepared in the intermediate process of Preparation Example 6 was dissolved in 3 ml of 1,2-dichloroethane. The solution was admixed with a 0.3 ml of 37% aqueous solution of formaldehyde and 470 mg (2.2 mmol) of sodium triacetoxyboronhydride, and stirred at ambient temperature for 1 hour.

Thereafter, the pH of the resultant solution was adjusted to pH8 with saturated aqueous solution of sodium hydrogencarbonate and extracted with methylene chloride. The separated methylene chloride phase was washed with saturated brine and dried over sodium sulfate anhydride. After solvent removal by distillation under a reduced pressure, 2.4 g of an oily substance was obtained. The oily substance was purified by using silica gel column (Merck Silica gel 60, 230-400 mesh, Merck Co., eluent composition: Hexane/AcOEt=5/1). This gave 450 mg of desired compound [I-12] as a clear oily substance. The yield was 86.3%.

Using the preparation procedures analogous to Preparation Examples from 1 to 7, compounds from I-1 to I-8, I-10 and I-11, from I-13 to I-19 and from I-22 to I-27 listed in Tables 1 to 4 were prepared. The following Tables 5 to 7 show the preparation procedures (corresponding to each procedure in Preparation Examples from 1 to 7) and the NMR data of each obtained compound. Note that although the NMR data of I-12 was obtained from the compound of Preparation Example 2, the NMR data of the compounds named I-12 obtained from Preparation Examples 6 and 7 are identical to those of Preparation Example 2.

Furthermore, in the following Formulation Examples and Test Examples, following compounds from (a) to (d) and from (m-1) to (m-3) were used as comparative compounds described in WO99/12902. Figure 2 illustrates the molecular structure of the compounds from (a) to (d) and from (m-1) to (m-3).

(a)
6-chloro-N-methyl-[2-methyl-3-(3-trifluoromethylphenyl) propyl]-3-pyridylmethylamine
(b)
6-chloro-N-methyl-[2-methyl-3-(3-trifluoromethoxyphenyl )propyl]-3-pyridylmethylamine
(c-1)
6-chloro-N-[3-(2,3-dichlorophenyl)-2-methylpropyl]-N-me thyl-3-pyridylmethylamine
(c-2)
6-chloro-N-[3-(2-chlorophenyl)-2-methylpropyl]-N-methyl -3-pyridylmethylamine

(c-3)
6-chloro-N-[3-(3-chlorophenyl)-2-methylpropyl]-N-methyl -3-pyridylmethylamine
(c-4)
6-chloro-N-[3-(3-fluorophenyl)-2-methylpropyl]-N-methyl -3-pyridylmethylamine
(d)
6-chloro-N-[2-methyl-3-(3,4-methylenedioxyphenyl)propyl ]-N-methyl-3-pyridylmethylamine
(m-1)
6-chloro-N-[3-(3,4-dimethylphenyl)-2-methylpropyl]-N-me thyl-3-pyridylmethylamine
(m-2)
N-[3-(4-t-butylphenyl)-2-methylpropyl]-6-chloro-N-methy 1-3-pyridylmethylamine
(m-3)
6-chloro-N-[3-(3-methoxyphenyl)-2-methylpropyl]-N-methy 1-3-pyridylmethylamine.

[Table 5]

| Compound No . Preparation Procedure a) | 1H-NMR (δ, ppm) |
|---|---|
| I-1 (Preparation Example 1) | 0.83 (d, 3H, J=6.4Hz), 1.90-2.06 (m, 1H), 2.19 (s, 3H) , 2.22 (d, 2H, J=2.0Hz), 2.41 (dd, 1H, J=13.2Hz, J=8.3Hz), 2.98 (dd, 1H, J=13.7Hz, J=4.9Hz), 3.48 (s, 2H), 7.30 (d, 1H, J=7.8Hz) , 7.95 (m, 2H), 7.64 (dd, 1H, J=8.3Hz,J=2.4Hz), 7.72 (m, 1H), 8.31 (d, 1H, J=2.0Hz) |
| I-2 (Preparation Example 1) | 0.85 (d, 3H, J=6.4Hz) , 2.01-2.15 (m, 1H), 2.18 (s, 3H), 2.26 (d, 2H, J=6.8Hz), 2.34-2.45 (m, 1H) 3.14-3.22 (m, 1H), 3.47 (s, 2H), 7.29 (d, 1H, J=8.3Hz), 7.43 (d, 1H, J=7.8Hz), 7.65 (dd, 1H, J=2.0, 8.3Hz) , 7.71 (d, 1H, J=7.8Hz) , 7.89 (s, 1H), 8.30 (d, 1H, J=2.0Hz) |
| I-3 (Preparation Example 1) | 0.85 (d, 3H, J=6.8Hz) , 2.02-2.15 (m, 1H), 2.18 (s, 3H), 2.26 (d, 2H, J=7.3Hz), 2.37-2.46 (m, 1H) 3.14-3.21 (m, 1H), 3.47 (s, 2H), 7.29 (d, 1H, J=8.3Hz), 7.57 (d, 2H, J=7.8Hz), 7.65 (dd, 1H, J=2.0, 8.3Hz) , 7.77 (d, 1H, J=7.8Hz) , 8.29 (d, 1H, J=2.0Hz) |
| I-4 (Preparation Example 1) | 0.85 (d, 3H, J=6.4Hz), 1.98-2.28 (m, 3H), 2.19 (s, 3H), 2.35-2.43 (m, 1H), 2.98 (dd, 1H, J=4.4, 13.7Hz), 3.48 (s, 2H) , 6. 68 (t, 1H, d=56.2Hz) , 7.30 (d, 1H, J=8.3Hz) , 7 . 48 (s, 1H), 7.52 (s, 1H), 7.61 (s, 1H), 7. 65 (dd, 1H, J=2.0, 8.3Hz), 8.32 (d, 1H, J=2.0Hz) |
| I-5 (Preparation Example 2) | 0.83 (d, 3H, J=6Hz), 1.77-3.10 (m, 5H), 2.13 (s, 3H), 3.38 (s, 2H), 7.07-7.43 (m, 4H), 7.53 (dd, 1H, J=2, 8Hz), 8.22 (d, 1H, J=2Hz) |
| I-6 (Preparation Example 1) | 0.80 (d, 3H, J=6Hz), 1.57-3.03 (m, 5H), 2.12 (s, 3H), 3.38 (s, 2H), 6.77-7.33 (m, 4H), 7.55 (dd, 1H, J=2, 8Hz), 8.18 (d, 1H, J=2Hz) |
| I-7 (Preparation Example 1) | 0.84 (d, 3H, J=6.4Hz), 2.00-2.06 (m, 1H), 2.16-2.31 (m, 3H) , 2.19 (s, 3H), 3.04 (dd, 1H, J=3.4, 13.7Hz) , 3.45 (dd, 2H, J=13.7, 23.4Hz) , 7.11-7.17 (m, 1H), 7.29 (d, 1H, J=8.3Hz), 7.34-7.48 (m, 2H) , 7. 66 (dd, 1H, J=2.0, 8.3Hz), 8.31 (d, 1H, J=2.0Hz) |
| I-8 (Preparation Example 2) | 0.82 (d, 3H, J=6Hz), 1.63-2.50 (m, 3H), 2.15 (s, 3H), 2.67-3.17 (m, 2H), 3.40 (s, 2H), 6.93-7.37 (m, 3H), 7.15 (d, 1H, J=8Hz), 7.53 (dd, 1H, J=2, 8Hz) , 8.20 (d, 1H, J=2Hz) |
| I-9 Preparation Example 1 | 0.82 (d, 3H, J=6Hz), 1.43-3.13 (m, 5H), 2.15 (s, 3H), 3.40 (s, 2H), 6.67-7.32 (m, 3H) , 7.32 (d, 1H, J=8Hz) , 7.53 (dd, 1H, J=2, 8Hz), 8.22 (d, 1H, J=2Hz) |

a) The Preparation Examples in the parentheses ( ) means that the compounds listed above were prepared by the preparation procedure according to Preparation Examples.

[Table 6]

| Compound No.<br>Preparation Procedure a) | 1H-NMR (δ, ppm) |
|---|---|
| I-10<br>(Preparation Example 1) | 0.85 (d, 3H, J=6.8Hz), 2.02-2-15 (m, 1H), 2.18 (s, 0.85 (d, 3H, J=6.8Hz), 2.02-2.15 (m, 1H), 2.18 (s, 3H), 2.26 (d, 2H, J=7.3Hz), 2.37-2.46 (m, 1H), 3.14-3.21 (m, 1H), 3.47 (s, 2H), 7.29 (d, 1H, J=8.3Hz), 7.57 (d, 2H, J=7.8Hz), 7.65 (dd, 1H, J=2.0, 8.3Hz), 7.77 (d, 1H, J=8.3Hz) , 8.29 (d, 1H, J=2.0Hz) |
| I-11<br>(Preparation Example 1) | 0.85 (d, 3H, J=6.8Hz), 1.90-2.06 (m, 1H), 2.17 (s, 3H) , 2.26 (d, 2H, J=6.4Hz), 2.41 (dd, 1H, J=15.1Hz, J=8.8Hz), 3.11 (dd, 1H, J=15.1Hz, J=2.0Hz), 3.40 (d, 1H, J=13.7Hz), 3.54 (d, 1H, J=13.7Hz), 7.29 (d, 1H, J=8.3Hz), 7.62 (dd, 1H, J=7.8Hz, J=2.4Hz), 8.30 (d, 1H, J=2.0) |
| I-12<br>Preparation Example 2 Preparation Example 6<br>Preparation Example 7 | 0.84 (d, 3H, J=6.4Hz), 2.00-2.06 (m, 1H), 2.16-2.31 (m, 3H) , 2.19 (s, 3H) , 3.04 (dd, 1H, J=3.4, 13.7Hz), 3.45 (dd, 1H, J=13.7, 23.4Hz) , 7.11-7.17 (m, 1H), 7.29 (d, 1H, J=8.3Hz) , 7.32-7.48 (m, 2H), 7. 66 (dd, 1H, J=2.0, 8.3Hz), 8.31 (d, 1H, J=2.0Hz) |
| I-13<br>(Preparation Example 2) | 0.84 (d, 3H, J=6.8Hz) , 2.10-2.30 (m, 3H) , 2 .18 (s, 3H), 2.80 (dd, 1H, J=4.9, 13.2Hz), 3.46 (s, 2H), 6.49 (t, 1H, J=74.2Hz), 7.02 (d, 2H, J=8.8Hz), 7.12 (d, 2H, J=8.8Hz), 7.29 (d, 1H, J=7.8Hz), 7. 65 (dd, 1H, J=2.4, 7.8Hz) , 8.30 (d, 1H, J=2.4Hz) |
| I-14<br>(Preparation Example 2) | 0.82 (d, 3H, J=6Hz), 1.45-3.05 (m, 5H), 2.13 (s, 3H) , 3.40 (s, 2H) , 4.90 (d, t, q, 1H, J=45, 6, 6Hz) , 7.03 (s, 4H), 7.18 (d, 1H, J=8Hz), 7.57 (dd, 1H, J=2, 8Hz), 8 .23 (d, 1H, J=2Hz) |
| I-15<br>(Preparation Example 1) | 0.82 (d, 3H, J=6Hz), 1.55-2.98 (m, 5H), 2.12 (s, 3H), 3.40 (s, 2H), 6.82-7.42 (m, 4H), 7.57 (dd, 1H, J=2, 8Hz), 8.23 (d, 1H, J=2Hz) |
| I-16<br>(Preparation Example 1) | 0.82 (d, 3H, J=6Hz), 1.47-2.97 (m, 5H), 2.13 (s, 3H), 3.40 (s, 2H), 6.80-7.40 (m, 4H), 7.58 (dd, 1H, J=2, 8Hz), 8.25 (d, 1H, J=2Hz) |
| I-17<br>Preparation Example 4 | 0.82 (d, 3H, J=6Hz), 1.93-3.10 (m, 5H), 2.15 (s, 3H) , 3.40 (s, 2H) , 6.67-7.42 (m, 3H) , 7.18 (d, 1H, J=8Hz) , 7.55 (dd, 1H, J=2, 8Hz) , 8.22 (d, 1H, J=2Hz) |
| I-18<br>(Preparation Example 1) | 0.88 (d, 3H, J=5.6Hz), 1.6-3.1 (m, 5H), 2.22 (s, 3H) , 3. 48 (s, 2H) , 6.8-7.4 (m, 4H) , 7.62 (dd, 1H, J=8.2, 2.4Hz), 8.32 (d, 1H, J=2.4Hz) |

a) The Preparation Examples in the parentheses ( ) means that the compounds listed above were prepared by the preparation procedure according to the Preparation Examples.

[Table 7]

| Compound No. Preparation Procedure a) | 1H-NMR (δ, ppm) |
|---|---|
| I-19<br>(Preparation Example 1) | 0.80 (d, 3H, J=5.6Hz), 1.7-3.0 (m, 5H), 2.13 (s, 3H), 3.42 (s, 2H), 6.9-7.3 (m, 4H), 7.60 (dd, 1H, J=7.6, 2.4Hz), 8.27 (d, 1H, J=2.4Hz) |

a) The Preparation Examples in the parentheses ( ) means that the compounds listed above were prepared by the preparation procedure according to the Preparation Examples.

[Table 7] (continued)

| Compound No. Preparation Procedure a) | 1H-NMR (δ, ppm) |
|---|---|
| I-20<br>Preparation Example 5 | 0.80 (d, 3H, J=6Hz), 1.37-3.03 (m, 5H), 2.13 (s, 3H), 3.40 (s, 2H), 6.47-6.90 (m, 2H), 7.18 (d, 1H, J=8Hz) , 7.55 (dd, 1H, J=2, 8Hz) , 8.23 (d, 1H, J=2Hz) |
| I-21<br>Preparation Example 3 | 0.82 (d, 3H, J=6Hz), 1.57-3.10 (m, 5H), 2.15 (s, 3H) , 3.40 (s, 2H) , 6.53-7.00 (m, 3H) , 7.20 (d, 1H, J=8Hz) , 7.58 (dd, 1H, J=2, 8Hz) , 8.25 (d, 1H, J=2Hz) |
| I-22<br>(Preparation Example 1) | 0.82 (d, 3H, J=5.6Hz), 1.6-2.4 (m, 4H),2.13 (s, 3H), 2.6-3.0 (m, 1H), 3.40 (s,. 2H), 7.04 (d, 2H, J=6.0Hz), 7.27 (d, 1H, J=7.9Hz), 7.62 (dd, 1H, J=7.9, 2.3Hz), 8.25 (d, 1H, J=2.3Hz) |
| I-23<br>(Preparation Example 1) | 0.85 (d, 3H, J=6.4Hz) , 1.90-2.05 (m, 3H) , 2.17 (s, 3H) , 2.25 (d, 2H, J=6.8Hz), 2 .35 (dd, 1H, J=11.0Hz, J=2.0Hz), 3.00 (dd, 1H, J=11.0Hz, J=4.4Hz), 3.41 (d, 1H, J=13.7Hz), 3.53 (d, 1H, J=13.7Hz), 7.30 (d, 1H, J=7.9Hz) , 7. 65 (dd, 1H, J=8.3Hz, J=2. 4Hz) , 8.31 (d, 1H, J=2.4) |
| I-24<br>(Preparation Example 1) | 0.82 (d, 3H, J=6Hz), 2.02-3.02 (m, 5H), 2.13 (s, 3H), 3.40 (s, 2H), 6.72-7.00 (m, 3H), 7.20 (d, 1H, J=8Hz) , 7.58 (dd, 1H, J=2, 8Hz) , 8.23 (d, 1H, J=2Hz) |
| I-25<br>(Preparation Example 1) | 0.84 (d, 3H, J=5.6Hz), 1.7-2.4 (m, 4H), 2.15 (s, 3H), 2.5-3.0 (m, 1H), 3.44 (s, 2H), 7.4 (m, 4H), 7.61 (dd, 1H, J=8.0, 2.4Hz) , 8.23 (d, 1H, J=2.4Hz) |
| I-26<br>(Preparation Example 2) | 0.78 (d, 3H, J=6Hz), 1.50-3.05 (m, 5H), 2.12 (s, 3H) , 3 .38 (s, 2H), 6.77-7.22 (m, 3H) , 7.17 (d, 1H, J=8Hz) , 7.55 (dd, 1H, J=2, 8Hz) , 8. 22 (d, 1H, J=2Hz) |
| I-27<br>(Preparation Example 2) | 0.82 (d, 3H, J=6Hz), 1.78-3.22 (m, 5H), 2.15 (s, 3H), 3.42 (s, 2H), 6.85-7.42 (m, 4H), 7.58 (dd, 1H, J=2, 8Hz), 8.27 (d, 1H, J=2Hz) |

a) The Preparation Examples in the parentheses ( ) means that the compounds listed above were prepared by the preparation procedure according to the Preparation Examples.

Formulation Example 1

(Dusting Powder Formulation)

Thefollowingingredientswere pulverized and blended to prepare a dusting powder.

| | (Parts by weight) |
|---|---|
| Compound (e.g., I-9) | 3 |
| Clay | 40 |
| Talc | 57 |

Formulation Example 2

(Wettable Powder Formulation)

Thefollowingingredientswere pulverized and blended to prepare wettable powder, and diluted with water for use.

| | (Parts by Weight) |
|---|---|
| Compound (e.g., I-12) | 50 |
| Lignin sulfonate | 5 |

(continued)

| | (Parts by Weight) |
|---|---|
| Alkylsulfonate | 3 |
| Diatomaceous earth | 42 |

Formulation Example 3

(Granule Formulation)

The following ingredients were uniformly blended, kneaded with water, processed in granular form using an extruder-type granulator and dried. This gave granules.

| | (Parts by Weight) |
|---|---|
| Compound (e.g., I-17) | 5 |
| Bentonite | 43 |
| Clay | 45 |
| Lignin sulfonate | 7 |

Formulation Example 4

(Emulsifiable Concentrate Formulation)

The following ingredients were equally blended and dissolved to prepare an emulsifiable concentrate.

| | (Parts by Weight) |
|---|---|
| Compound (e.g., I-21) | 20 |
| Polyoxyethylenealkyl allyl ether | 10 |
| Polyoxyethylene sorbitan monolaurate | 3 |
| Xylene | 67 |

According to the procedures analogous to the Formulation Examples 1 to 4, four types of preparations '(dusting powder, wettable powder, granule and emulsifiable concentrate) were prepared using each compound of the present invention from I-1 to I-27 and each comparative compound from (a) to (d) and from (m-1) to (m-3).

Test Example 1

(Test of Control Effect on Wheat Powdery Mildew)

Wettable powders were prepared from compounds of the present invention I-1 to I-27 and comparative compounds (a) to (d) , (m-1) and (m-3). The obtained wettable powder was diluted and suspended with water to provide the predetermined concentration (125 mg/l), and was sprinkled onto seedlings of wheat (variety: "Nourin 64") at the first to the second leaf period which were cultivated using square plastic pots (size: 6.4 cm x 6.4 cm) in a rate of 100 liters/10 a (ares). After drying the sprinkled leaves with air, a suspension of wheat powdery mildew spores taken from infected leaves was inoculated by spraying onto the sprinkled leaves. They were supervised in a green house (temperature: 20 to 24°C, relative humidity: 20 to 70 RH). On the 9th to 14th day after the inoculation, the infection degree was examined according to the following scale and the control value of each compound was calculated by the following calculation equation 1.

(Examination Scale)

| <Infection Degree> | <Severity> |
|---|---|
| 0 | No infection |
| 0.5 | Lesion area proportion: less than 1% |
| 1 | Lesion area proportion: more than 1% and less than 5% |
| 2 | Lesion area proportion: more than 5% and less than 10% |
| 3 | Lesion area proportion: more than 10% and less than 30% |
| 4 | Lesion area proportion: more than 30% and less than 50% |
| 5 | Lesion area proportion: more than 50% |

(Equation 1)

Control value = (1 - infection degree of treated zone/infection degree of untreated zone) x 100 (%)

The control values of each compound are shown in Table 8.

[Table 8]

| Compound No. | Control Value |
|---|---|
| I-1 | 100 |
| I-2 | 100 |
| I-3 | 90 |
| I-4 | 100 |
| I-5 | 100 |
| I-6 | 100 |
| I-7 | 100 |
| I-8 | 100 |
| I-9 | 100 |
| I-10 | 100 |
| I-11 | 90 |
| (a) | 70 |
| | |
| I-12 | 100 |
| I-13 | 100 |
| I-14 | 100 |
| I-15 | 100 |
| I-16 | 100 |
| I-17 | 100 |
| I-18 | 100 |
| I-19 | 100 |
| I-20 | 100 |
| (b) | 80 |

[Table 8] (continued)

| Compound No. | Control Value |
|---|---|
| | |
| I-21 | 100 |
| I-22 | 100 |
| I-23 | 100 |
| (c-1) | 40 |
| (c-2) | 40 |
| (c-3) | 50 |
| (c-4) | 10 |
| | |
| I-24 | 100 |
| I-25 | 100 |
| I-26 | 100 |
| I-27 | 100 |
| (d) | 10 |
| | |
| (m-1) | 10 |
| (m-3) | 0 |

As described above, it is confirmed that if the compounds I-1 to 27 according to the present invention are used, a high control value can be obtained for each case, and a significantly excellent control effect is exerted to wheat powdery mildew.

Test Example 2

(Test of Control Effect on Cucumber Powdery Mildew)

The wettable powders formulated by using each of the compounds I-1 to 27 according to the present invention and the comparative compounds (a) to (d), (m-1) and (m-3) were each diluted and suspended with water to a predetermined concentration (100 mg/l), the suspension was sprinkled over the first to the second leaf period cucumbers (variety: "Sagamihanpakufushinari") cultivated by using a square plastic pot (size: 6. 4 cm x 6. 4 cm) in a rate of 100 liters/10 a (ares). After the sprinkled leaves were air-dried, the spores were inoculated over the leaves by sprinkling the same with a brush from an infected leaf, a disease was contracted in a glass house (temperature: 20 to 24°C, relative humidity: 20 to 70 RH). On the 9th to 14th day after the inoculation, the infection degree was examined according to the following scale and the control value of each compound was calculated by the following calculation equation 2:

(Examination Scale)

| <Infection Degree> | <Severity> |
|---|---|
| 0 | No infection |
| 0.5 | Lesion area proportion: less than 5% |
| 1 | Lesion area proportion: more than 5% and less than 10% |
| 2 | Lesion area proportion: more than 10% and less than 30% |
| 3 | Lesion area proportion: more than 30% and less than 50% |
| 4 | Lesion area proportion: more than 50% |

(continued)

| <Infection Degree> | <Severity> |
|---|---|
| | and less than 70% |
| 5 | Lesion area proportion: more than 70% |

(Equation 2)

Control value = (1 - infection degree of treated zone/infection degree of untreated zone) x 100 (%)

The control values of each compound are shown in Table 9.

[Table 9]

| Compound No. | Control Value |
|---|---|
| I-1 | 100 |
| I-2 | 100 |
| I-3 | 100 |
| I-4 | 80 |
| I-5 | 100 |
| I-6 | 100 |
| I-7 | 90 |
| I-8 | 100 |
| I-9 | 100 |
| I-10 | 100 |
| I-11 | 100 |
| (a) | 50 |
| | |
| I-12 | 100 |
| I-13 | 100 |
| I-14 | 100 |
| I-15 | 100 |
| I-16 | 100 |
| I-17 | 100 |
| I-18 | 100 |
| I-19 | 100 |
| I-20 | 100 |
| (b) | 60 |
| | |
| I-21 | 100 |
| I-22 | 100 |
| I-23 | 70 |
| (c-1) | 0 |
| (c-2) | 0 |
| (c-3) | 0 |
| (c-4) | 0 |
| | |
| I-24 | 100 |
| I-25 | 100 |

[Table 9] (continued)

| Compound No. | Control Value |
|---|---|
| I-26 | 100 |
| I-27 | 100 |
| (d) | 0 |
| | |
| (m-1) | 10 |
| (m-3) | 0 |

As described above, it is confirmed that if the compounds I-1 to 27 according to the present invention are used, a high control value can be obtained for each case, and a significantly excellent control effect is exerted to cucumber powdery mildew.

Test Example 3

(AntifungalActivity Test againstVariousPathogenic Fungi)

For each of the compounds I-1 to 27 of the present invention and the comparative compounds (a) to (d) , (m-1) and (m-3), 20 mg was weighed and dissolved in 2 ml of dimethylsulfoxide, and a double dilution row in 10 steps was prepared by further using dimethylsulfoxide. A 0. 6 ml portion of these solutions was each added to 60 ml of a PDA culture medium (potato-dextrose-agar culture medium) at about 60°C, they were well mixed in a 100 ml Erlenmeyer flask, the solution was poured into a schale and was solidified, the plate culture media containing the compounds according to the present invention with final concentrations of 100, 50.0, 25.0, 12.5, 6. 3, 3.2, 1. 6, 0.8, 0.4 and 0.2 mg/l were prepared.

On the other hand, the specimen bacteria cultured on the plate culture media beforehand was punched by a 4mm diameter cork borer, and was inoculated on the plate culture media containing the aforementioned chemicals. After the inoculation, the bacteria were cultured at the optimum growth temperature of each bacterium for 1 to 7 days (for this optimum growth temperature, LIST OF CULTURES 1996 Microorganisms 10th edition published by Fermentation Research Institute (Foundation) can be referred to.), and the growth of the bacteria was measuredby the diameter of a colony. The growth extent of the bacteria obtained on the plate culture media was compared with that of the bacteria in the chemical free area to find hypha extension inhibition rate with the following calculation equation 3.

(Equation 3)

$$R = 100 (dc - dt) / dc$$

[wherein each symbol is shown as follows: R = Hypha extension inhibition rate (%), dc = Diameter of colony on no-treated plate, dt = Diameter of colony on chemical-treated plate.]

The results obtained above was evaluated in 5 steps in accordance with the following examination scale.

<Growth Inhibition>

5 : Hypha extension inhibition rate of 90% or more
4 : Hypha extension inhibition rate of from 70% to less than 90%
3 : Hypha extension inhibition rate of from 40% to less than 70%
2 : Hypha extension inhibition rate of from 20% to less than 40%
1 : Hypha extension inhibition rate of less than 20%

The obtained evaluation results are shown in Table 10. In addition, the abbreviations in Table 10 are as follows:

B.c : Botrytis cinerea
M.n : Microdochium nivale

L.n : Leptosphaeria nodorum
R.s : Rhynchosporium secalis
P.g : Pyrenophora graminea
P.o : Pyricularia oryzae
V.i : Venturia inaequalis
P.h : Pseudocercosporella herpotrichoides
S.t : Septoria tritici

[Table 10]

| Compound | Specimen bacterium (Concentration [ppm]) | | | | | | | | |
|---|---|---|---|---|---|---|---|---|---|
| | B.c (1.6) | M.n (3.2) | L.n (0.8) | R.s (100) | P.g (12.5) | P.o (12.5) | V.i (3.2) | P.h (25) | S.t (100) |
| I-1 | 4 | - | - | - | - | - | - | - | - |
| I-2 | 4 | - | - | - | - | - | - | - | - |
| I-3 | 4 | - | - | - | - | - | - | - | - |
| I-4 | 4 | - | - | - | - | - | - | - | - |
| I-5 | 4 | - | - | - | - | - | - | - | - |
| I-6 | 4 | - | - | - | - | - | - | - | - |
| I-7 | 4 | - | - | - | - | - | - | - | - |
| I-8 | 4 | - | - | - | - | - | - | - | 4 |
| I-9 | 4 | - | - | - | - | - | - | - | 4 |
| I-10 | 5 | 4 | 4 | 4 | 4 | 4 | 4 | 5 | 4 |
| I-11 | 4 | - | - | - | - | - | - | - | - |
| (a) | 2 | - | - | - | - | - | - | - | - |
| | | | | | | | | | |
| I-12 | 4 | 5 | 5 | 5 | 4 | 4 | 4 | 5 | 4 |
| I-13 | 4 | - | - | - | - | - | - | - | - |
| I-14 | 4 | - | - | - | - | - | - | - | - |
| I-15 | 4 | - | - | - | - | - | - | - | - |
| I-16 | 4 | - | 5 | - | - | - | - | - | - |
| I-17 | 5 | 5 | 5 | 5 | 4 | 4 | 4 | 5 | 4 |
| I-18 | 5 | - | - | - | - | - | - | - | - |
| I-19 | 4 | - | - | - | - | - | - | - | - |
| I-20 | 5 | 4 | 5 | 4 | 4 | 4 | 4 | 5 | 4 |
| (b) | 3 | - | - | - | - | - | - | - | - |
| | | | | | | | | | |
| I-21 | 5 | 4 | 5 | 5 | 5 | 4 | 4 | 5 | 4 |
| I-22 | 4 | - | - | - | - | - | - | - | - |
| I-23 | 4 | - | - | - | - | - | - | - | - |
| (c-1) | 2 | - | - | - | - | - | - | - | - |
| (c-2) | 2 | 2 | 3 | 2 | 2 | 3 | 2 | - | - |
| (c-3) | 2 | - | - | - | - | - | - | - | - |
| (c-4) | 1 | 2 | 3 | 2 | 2 | 3 | 2 | - | - |
| | | | | | | | | | |
| I-24 | 4 | 4 | 5 | 5 | 5 | 4 | 4 | 5 | 4 |
| I-25 | 4 | - | - | - | - | - | - | - | - |
| I-26 | 4 | - | - | - | - | - | - | - | - |
| I-27 | 4 | - | - | - | - | - | - | - | - |

[Table 10] (continued)

| Compound | Specimen bacterium (Concentration [ppm]) | | | | | | | | |
|---|---|---|---|---|---|---|---|---|---|
| | B.c (1.6) | M.n (3.2) | L.n (0.8) | R.s (100) | P.g (12.5) | P.o (12.5) | V.i (3.2) | P.h (25) | S.t (100) |
| (d) | 1 | - | - | - | - | - | - | - | - |
| | | | | | | | | | |
| (m-1) | 2 | | | | | | | | |
| (m-3) | 1 | - | - | - | - | - | - | - | - |

As described above, it is confirmed that if the compounds I-1 to 27 according to the present invention are used, a high growth inhibition can be obtained for each case, and asignificantly excellent antifungalactivityis exerted to the various pathogenic fungi.

<u>Test Example 4</u>

(Permeable Mobility Fungicidal Effect Test in Leaf Section of Wheat Powdery Mildew)

In the test example 4, permeable mobility fungicidal effect test was performedon the leaf section of wheat powdery mildew with regard to the compounds I-1 to 27 according to the present invention and the comparative compounds (a) to (d), and (m-1) to (m-3) with the method described later.

<Test method>

The compound in the form of a wettable powder obtained by Formulation 2 or the like was diluted and suspended with water to a predetermined concentration (200mg/l), and 0.5 µl of the suspension was applied to a 2 to 3 cm leaf portion from the root of the three leaves of the 4th to 4.5th leaf period wheat (variety: Abukumawase) cultured by using a square plastic pot (size: 6.4 cm x 6.4 cm). After these wheat leaves were subject to still standing in a glass house (temperature: 20 to 24C, relative humidity: 20 to 70 RH) for one day, the spores of wheat powdery mildew were inoculated over the leaves by sprinkling the same with a brush from an infected leaf, a disease was contracted in the same glass house. The length of a non-contracted area generated from the applied portion to the direction of the leaf top was measured, and a relative mobility to the compound (m-2) was calculated with the following calculation equation 3 by using the comparative compound (m-2) as a standard compound.

(Equation 4)

Relative mobility = (Length of non-contracted area in each chemical section (cm)/Length of non-contracted area of compound (m-2) (cm))

The results obtained by the aforementioned test are shown in Table 11.

[Table 11]

| Compound | Relative Mobility |
|---|---|
| I-1 | 3 |
| I-2 | 3 |
| I-3 | 3 |
| I-4 | 3 |
| I-5 | 3 |
| I-6 | 5 |

[Table 11] (continued)

| Compound | Relative Mobility |
|---|---|
| I-7 | 5 |
| I-8 | 5 |
| I-9 | 3 |
| I-10 | 3 |
| I-11 | 5 |
| (a) | 1 |
| | |
| I-12 | 10 |
| I-13 | 9 |
| I-14 | 7 |
| I-15 | 9 |
| I-16 | 9 |
| I-17 | 10 |
| I-18 | 8 |
| I-19 | 8 |
| I-20 | 9 |
| (b) | 3 |
| | |
| I-21 | 9 |
| I-22 | 3 |
| I-23 | 4 |
| (c-1) | 1 |
| (c-2) | 1 |
| (c-3) | 1 |
| (c-4) | 1 |
| | |
| I-24 | 6 |
| I-25 | 3 |
| I-26 | 3 |
| I-27 | 3 |
| (d) | 1 |
| | |
| (m-1) | 1 |
| (m-2) | 1 |
| (m-3) | 1 |

As mentioned above, it is confirmed that if the compounds I-1 to 27 according to the present invention are used, although the compound is unevenly applied at the spray timing, a significantly excellent fungicidal and control effect is exerted since the chemical is permeably transferred from a region where the compound is peripherally applied.

## Industrial Applicability

As described above, it is possible to obtain a fungicide which is suitably used for horticulture or the like, and has a higher fungicidal activity to pathogenic bacteria of plant diseases, as a result, has a significantly excellent control effect to the plant diseases, and is of low toxicity to men and animals and of high safety in handling.

## Claims

1. A 6-chloro-3-pyridylmethylpropylamine derivative of the following formula (I):

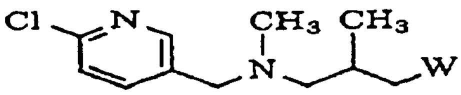

(I)

or an acid addition salt thereof;

wherein W represents a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group, 3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group, 2,3,5,6-tetrafluoro-4-trifluoromethylphenyl group, 4-trifluoromethoxyphenyl group, 4-difluoromethoxyphenyl group, 4-(1,1,2,3,3,3-hexafluoropropoxy)phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group, 3,5-difluoro-4-trifluoromethoxyphenyl group, 3-chloro-5-fluorophenyl group, 3,5-dichloro-4-fluorophenyl group, pentafluorophenyl group, 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl and 2,2,4,4-tetrafluoro-4H-1,3-benzodioxin-6-yl.

2. The 6-chloro-3-pyridylmethylpropylamine derivative or the acid addition salt thereof according to claim 1, wherein W in the general formula (I) is a group selected from the group consisting of 3,5-bistrifluoromethylphenyl group, 2,4-bistrifluoromethylphenyl group, 2,5-bistrifluoromethylphenyl group, 3-difluoromethyl-5-trifluoromethylphenyl group, 3-chloro-5-trifluoromethylphenyl group, 3-fluoro-5-trifluoromethylphenyl group, 2-fluoro-3-trifluoromethylphenyl group, 2-fluoro-4-trifluoromethylphenyl group, 3-fluoro-4-trifluoromethylphenyl group, 2,3-difluoro-4-trifluoromethylphenyl group and 2,3,5,6-tetrafluoro-4-trifluoromethylphenyl group.

3. The 6-chloro-3-pyridylmethylpropylamine derivative or the acid addition salt thereof according to claim 1, wherein W in the general formula (I) is a group selected from the group consisting of 4-trifluoromethoxyphenyl group, 4-difluoromethoxyphenyl group, 4-(1,1,2,3,3,3-hexafluoropropoxy)phenyl group, 4-chloro-3-trifluoromethoxyphenyl group, 4-fluoro-3-trifluoromethoxyphenyl group, 3-fluoro-4-trifluoromethoxyphenyl group, 3-chloro-4-trifluoromethoxyphenyl group, 3-chloro-4-bromodifluoromethoxyphenyl group and 3,5-difluoro-4-trifluoromethoxyphenyl group.

4. The 6-chloro-3-pyridylmethylpropylamine derivative or the acid addition salt thereof according to claim 1, wherein W in the general formula (I) is a group selected from the group consisting of 3-chloro-5-fluorophenyl group, 3,5-dichloro-4-fluorophenyl group and pentafluorophenyl group.

5. The 6-chloro-3-pyridylmethylpropylamine derivative or the acid addition salt thereof according to claim 1, wherein W in the general formula (I) is a group selected from the group consisting of 2,2-difluoro-1,3-benzodioxol-5-yl group, 2,2-dichloro-1,3-benzodioxol-5-yl group, 2,2,3,3-tetrafluoro-2,3-dihydro-1,4-benzodioxin-6-yl group and 2,2,4,4-tetratluoro-4H-1,3-benzodioxin-6-yl group.

## Fig.1A

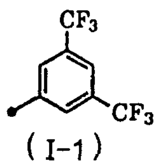
(I-1)

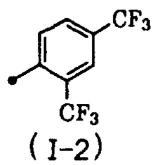
(I-2)

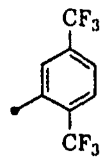
(I-3)

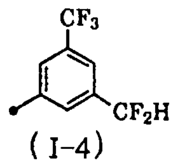
(I-4)

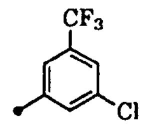
(I-5)

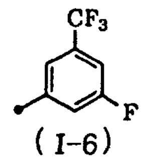
(I-6)

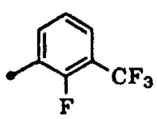
(I-7)

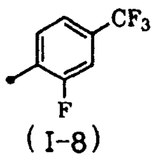
(I-8)

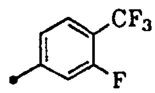
(I-9)

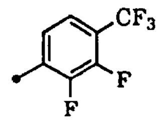
(I-10)

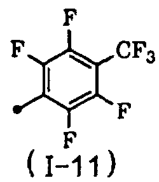
(I-11)

## Fig.1B

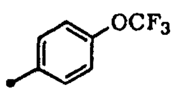
(I-12)

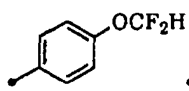
(I-13)

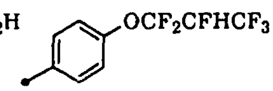
(I-14)

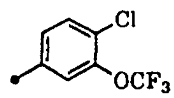
(I-15)

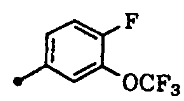
(I-16)

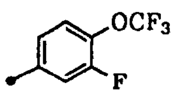
(I-17)

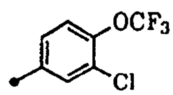
(I-18)

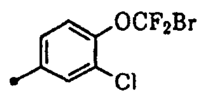
(I-19)

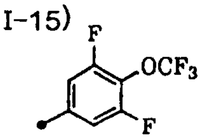
(I-20)

## Fig.1C

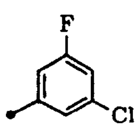
(I-21)

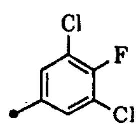
(I-22)

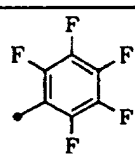
(I-23)

## Fig.1D

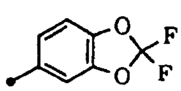
(I-24)

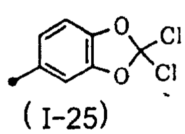
(I-25)

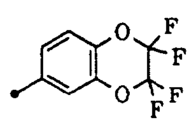
(I-26)

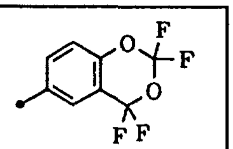
(I-27)

# Fig.2

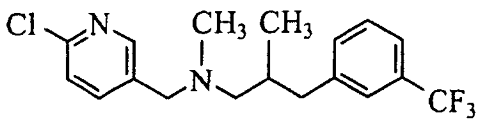

(a)

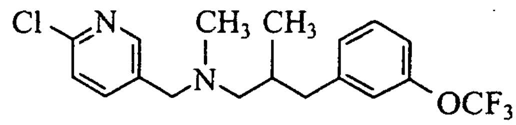

(b)

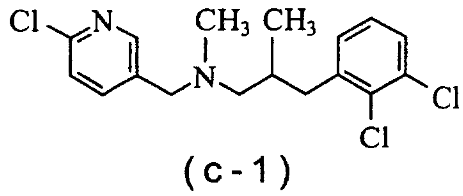

(c-1)

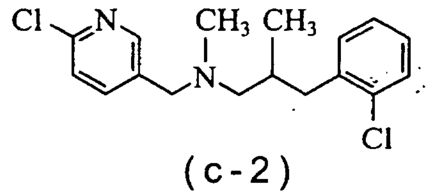

(c-2)

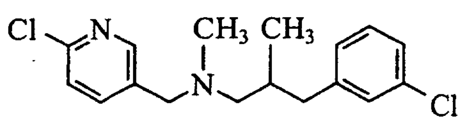

(c-3)

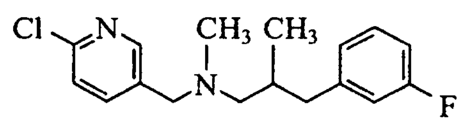

(c-4)

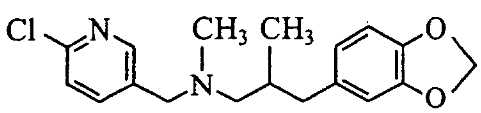

(d)

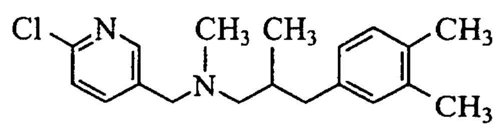

(m-1)

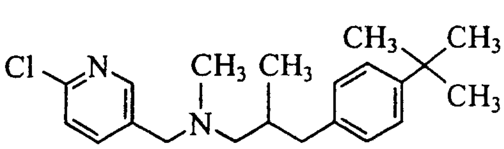

(m-2)

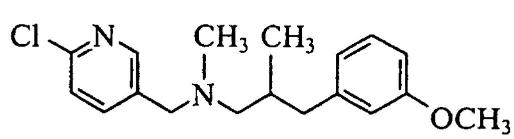

(m-3)

## INTERNATIONAL SEARCH REPORT

International application No.
PCT/JP01/01866

A. CLASSIFICATION OF SUBJECT MATTER
Int.Cl[7] C07D213/61, C07D405/12, A01N43/40, A01N47/00

According to International Patent Classification (IPC) or to both national classification and IPC

B. FIELDS SEARCHED

Minimum documentation searched (classification system followed by classification symbols)
Int.Cl[7] C07D213/00-61, C07D405/00-12, A01N43/00-40, A01N47/00

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| X | WO, 99/12902, A1 (Kureha Chemical Industry Co., Ltd.), 18 March, 1999 (18.03.99), entire description; especially compound Nos. I-81, I-84, I-89, I-91, etc. & EP, 1020441, A1 | 1-3 |
| A | US, 4552960, A (ELI LILLY AND COMPANY), 12 November, 1985 (12.11.85), entire description & JP, 60-13760, A | 1-3 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 June, 2001 (11.06.01) | 19 June, 2001 (19.06.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

International application No.
PCT/JP01/01866

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The compounds set forth in claims 1-3 have as the fundamental skeleton 6-chloro-N-methyl-[2-methyl-3-(substituted phenyl) propyl]-3-pyridylmethylamine in common. However, this fundamental skeleton itself is publicly known as disclosed in documents cited in the international search report, page 2, column C. Therefore, the compounds set forth in claims 1-3 are not considered in the sight of the prior art as having a common technical feature.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)